# EUROPÄISCHE PATENTSCHRIFT

(11) **EP 1 849 439 B1**
(45) Veröffentlichungstag und Bekanntmachung des Hinweises auf die Patenterteilung: **04.06.2014**
(21) Anmeldenummer: 07007845.6
(22) Anmeldetag: 18.04.2007
(51) Int. Cl.: A61F 2/64

(54) **Knieprothesengelenk**
Jointed knee prosthesis
Prothèse d'articulation du genou

(30) Priorität: 28.04.2006 DE 102006019858
(43) Veröffentlichungstag der Anmeldung: 31.10.2007
(73) Patentinhaber: Bauerfeind AG, 07937 Zeulenroda (DE)
(72) Erfinder: Reinhardt, Holger, 47906 Kempen (DE)
(74) Vertreter: Bardehle, Heinz

(56) Entgegenhaltungen:
- EP-A- 0 439 028
- EP-A- 0 672 398
- EP-A- 0 713 689
- DE-C- 448 069
- DE-U1- 8 910 199

## Beschreibung

Die Erfindung bezieht sich auf ein Knieprothesengelenk mit vier Achsbolzen zur Verbindung von vier Gelenkgliedern, die jeweils an ihren Enden in je einem Achsbolzen gelagert sind, wobei zwei gegenüberliegende, quer verlaufende Gelenkglieder einerseits mit einem Prothesenschaft und andererseits mit einem Prothesenfluß verbindbar sind und die beiden anderen Gelenkglieder als Längsgelenkglieder jeweils in die Standlage und der Beugelage im Wesentlichen aus einer der Parallellage angenäherten Winkellage in eine demgegenüber stärkere Schräglage zueinander verschwenkbar sind.

Ein derartiges Prothesengelenk ist in der DE 40 04 988 A1 veröffentlicht. Um bei diesem Knieprothesengelenk einerseits die Standsicherheit und andererseits die Erleichterung der Kniebeugung zu verbessern, ist der eine Achsbolzen, der in dem mit dem Prothesenfluß verbundenen Gelenkglied angeordnet ist, in einem Langloch gelagert und in diesem durch Anziehen einer Schraube feststellbar, wodurch bei im Effekt verlängerten Abstand zwischen den Achsbolzen dieses Gelenkgliedes das Abbeugen der Prothese erleichtert und im Falle der Verkürzung der Abstand der betreffenden Achsbolzen durch entsprechende Verschiebung in dem Langloch die Standsicherheit erhöht wird. Dabei hat sich gezeigt, dass einerseits die dauernde sichere Feststellung des betreffenden in dem Langloch verschiebbaren Achsbolzens nicht immer gewährleistet werden kann und andererseits der Achsbolzen in dem Langloch wegen seiner im Prinzip nur linienhaften Berührung mit der Innenfläche des Langlochs die notwendige Stabilität der jeweiligen Lage des Achsbolzens nicht gewährleistet ist.

Aus der DE 448 069 ist weiterhin ein Knieprothesengelenk bekannt, bei dem zwei Längsgelenkglieder vorgesehen sind, von denen das eine im Bereich des Prothesenschaftes auf einem in einer Längsnut geführten Schiebestein gelagert ist, der das eine Lager des einen Längsgelenkgliedes trägt. Mit der Verschiebung des Lagersteins in der Nut ergibt sich in Bezug auf ein zweites Längsgelenkglied eine veränderte Lagerstelle, wobei der Schiebestein in der jeweils eingenommenen Lage durch Stellschrauben festgehalten werden kann. Hierdurch wird eine Verlängerung oder Verkürzung der Prothese erzielt, was beim Beugen des Knies und beim Sitzen von Vorteil sein soll.

Der Erfindung liegt daher die Aufgabe zugrunde, den beweglichen Achsbolzen in dem mit dem Prothesenfuß verbindbaren Gelenkglied so zu lagern, dass dieser stets eine große Gegenlagerfläche findet und andererseits die Variationsmöglichkeit der Lage des Achsbolzen erweitert wird. Erfindungsgemäß geschieht dies dadurch, dass mindestens einer der Achsbolzen des mit dem Prothesenfuß verbundenen Gelenkgliedes als verdrehbarer, feststellbarer Exzenter ausgebildet ist, bei dessen Verdrehung das betreffende Längsgelenkglied zu dem Gelenkglied sich verschiebt und damit den Abstand zwischen den die beiden quer verlaufenden Gelenkglieder tragenden Achsbolzen ändert.

Durch Ausbildung des Achsbolzens als Exzenter ergibt sich eine großflächigere Lagerung für den Achsbolzen, die in der Lage ist, entsprechende Druckkräfte ohne weiteres aufzunehmen. Außerdem bietet der Exzenter den wesentlichen Vorteil, dass seine Lageveränderung durch seine Verdrehung über 360° möglich ist, also neben der Achsverkürzung direkt auch noch eine um 90° dazu verlaufende Verlagerung ermöglicht, was weiterhin der Anpassung des Knieprothesengelenks an den Träger von Vorteil ist, da damit gegebenenfalls in gewissem Umfang auf dessen Größe bzw. Längenveränderung aufgrund verschiedener Schuhhöhen Rücksicht genommen werden kann. Diese Anpassung ist insbesondere in einer Anfangsphase der Benutzung des Knieprothesengelenks, also insbesondere kurz nach einer Operation, von besonderer Bedeutung, da in dieser Phase der Träger erfahrungsgemäß zunächst eine hohe Standsicherheit sucht, nach deren Erreichen er dann langsam dazu übergeht, mit seiner Prothese auch zu gehen, wozu er stufenlos das Knieprothesengelenk an seine jeweilige auch psychisch bedingte Situation einstellen bzw. einstellen lassen kann. Je aktiver der Patient ist, um so dynamischer kann das Knieprothesengelenk eingestellt werden.

Es ist darüber hinaus auch möglich, mehrere Achsbolzen als Exzenter auszubilden. Hierdurch wird die Flexibilität der Anpassung des Knieprothesengelenks an die gegebene Situation des Trägers der Prothese bzw. an gewisse durch Kleidungsstücke oder Schuhe gegebene Änderungen erhöht.

In den Figuren sind Ausführungsbeispiele der Erfindung dargestellt. Es zeigen:
- Figur 1: eine perspektivische Ansicht des Knieprothesengelenks;
- Figur 2: das gleiche Knieprothesengelenk in Seitensicht;
- Figur 3: einen Schnitt durch den Exzenter in einem Achsbolzen;
- Figur 4: das Knieprothesengelenk mit seinem von der Einstellung des Exzenters abhängigen Momentandrehpunkt;
- Figuren 5a bis 5d: vier Stellungen des Exzenters;
- Figur 6: ein Knieprothesengelenk mit drei als Exzenter ausgebildeten Achsbolzen.

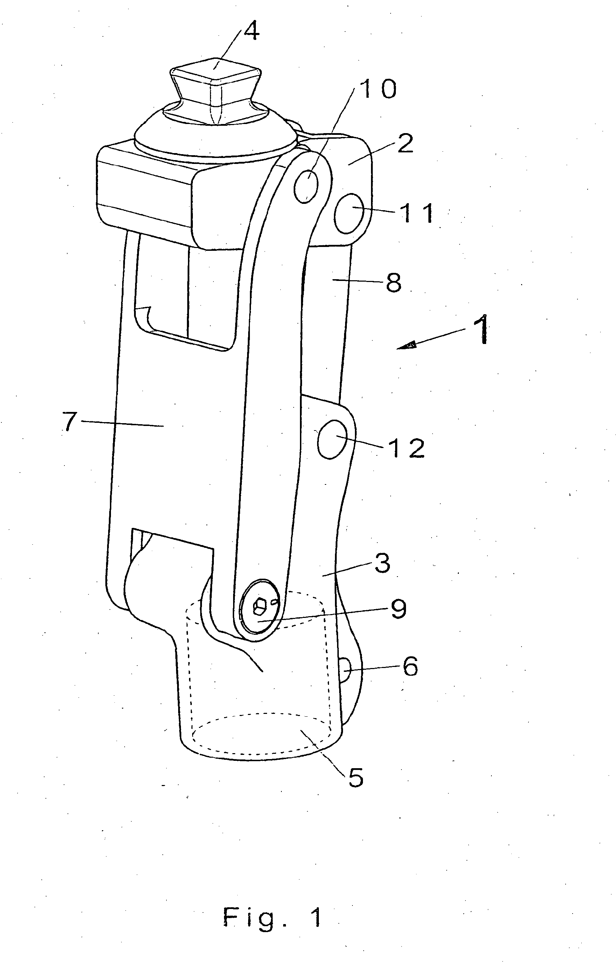

Das in der Figur 1 dargestellte Knieprothesengelenk 1 weist einerseits das Gelenkglied 2 zur Verbindung mit einem nicht dargestellten Prothesenschaft und das Gelenkglied 3 zur Verbindung mit einem nicht dargestellten Prothesenfuß auf. Für die Verbindung mit dem Prothesenschaft dient der Vierkantpyramidenadapter 4, auf den der Prothesenschaft in bekannter Weise aufgeschraubt wird. Zur Verbindung des anderen Gelenkgliedes 3 mit einem Prothesenfuß ist das Gelenkglied 3 mit einer Bohrung 5 (gestrichelt gezeichnet) versehen, die in bekannter Weise eine Verschraubung 6 aufweist und einen von dem Prothesenfuß wegragenden Stumpf aufnimmt, der mit der Verschraubung 6 festklemmbar ist.

Die beiden Gelenkglieder 2 und 3 sind mit den beiden Längsgelenkgliedern 7 und 8 verbunden, die jeweils an ihren Enden Achsbolzen 9, 10, 11 und 12 tragen. Über diese Achsbolzen sind die Längsgelenkglieder 7 und 8 mit den quer verlaufenden Gelenkgliedern 2 und 3 verbunden. Damit wird durch diesen Aufbau ein Knieprothesengelenk 1 gebildet, wie es prinzipiell in vielen Knieprothesengelenken verwendet wird (siehe auch die oben erwähnte DE 40 04 988 A1.

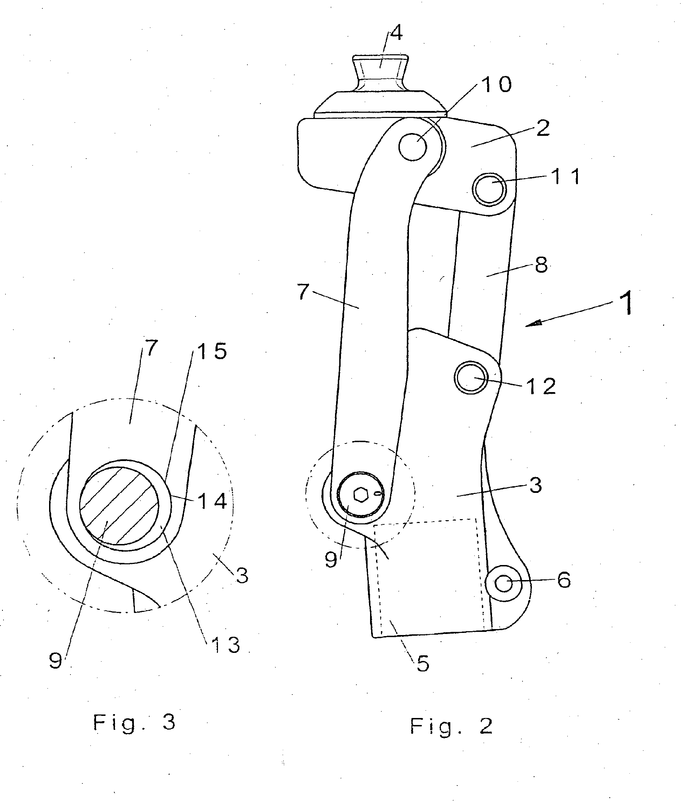

Die Besonderheit des in der Figur 1 dargestellten Knieprothesengelenks besteht darin, dass der Achsbolzen 9 als Exzenter ausgebildet ist, auf dessen Gestaltung und Wirkung im Zusammenhang mit der Figur 3 näher eingegangen wird.

Es sei zunächst auf die Figur 2 verwiesen, die das Knieprothesengelenk 1 gemäß Figur 1 in Seitensicht darstellt.

Für die Wirksamkeit des dargestellten Knieprothesengelenks 1 ist, wie eingangs dargelegt, der Abstand zwischen den Achsbolzen 9 und 12 wesentlich, da dieser Abstand einerseits die Standsicherheit und andererseits die Erleichterung der Abbiegefähigkeit fördert. Hierzu ist der Achsbolzen 9 als Exzenter 13 ausgebildet, der in der Figur 3 im Schnitt dargestellt ist. Gemäß Figur 3 ist der Exzenter 13 auf dem Achsbolzen 9 angebracht bzw. wächst aus diesem heraus und bildet mit seiner Außenfläche die Lagerfläche zur Bohrung 15 in dem Längsgelenkglied 7. Je nach Verdrehung des Achsbolzens 9 und damit des Exzenters 13 wandert dieser stufenlos in der Bohrung 15 und legt sich jeweils an eine andere Stelle der Bohrung 15, womit relativ zu dem Gelenkglied 3 das Längsgelenkglied 7 eine entsprechend andere Lage erhält.

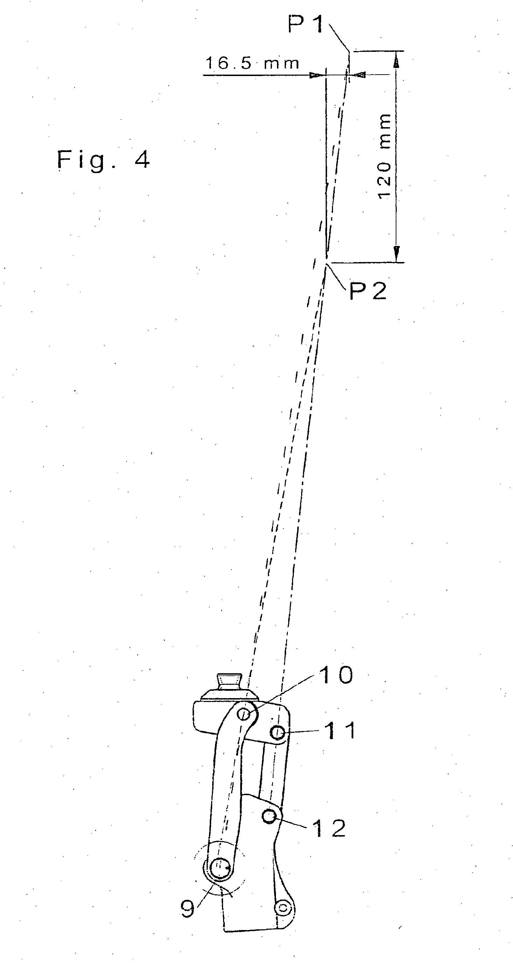

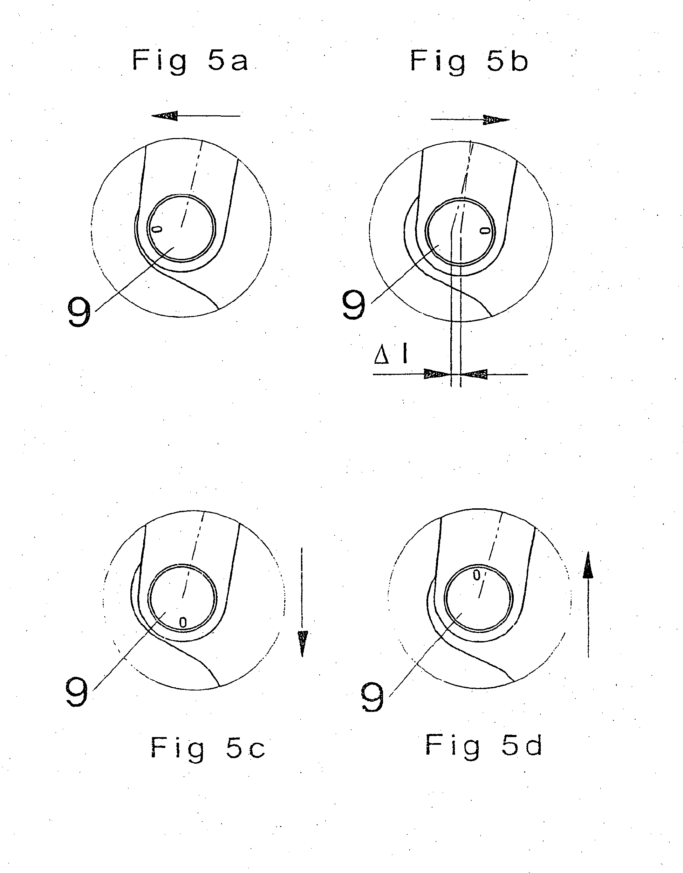

Die Folgen dieser Lageveränderung des Exzenters 13 sind in der Figur 4 dargestellt, die sich an die in der DE 40 04 988 A1 gegebene Darstellungsweise anlehnt (siehe dort Figur 7). In der Figur 4 sind zwei extreme Momentandrehpunkte P1 und P2 eingetragen, von denen der Momentandrehpunkt P1 für eine relativ kurze Entfernung der beiden Achsbolzen 9 und 12 gilt, während der Momentandrehpunkt P2 für die größte Entfernung zwischen den beiden Achsbolzen 9 und 12 gültig ist. Hierzu gehören die in Figur 5b (Momentandrehpunkt P1) und Figur 5a (Momentandrehpunkt P2) im einzelnen dargestellten Lagen des Exzenters 13 bzw. des ihn tragenden Achsbolzens 9.

Die in den Figuren 5c und 5d dargestellten Stellungen des Achsbolzens 9 und damit des Exzenters 13 repräsentieren jeweils eine Lage des Achsbolzens 9, in dem dieser jeweils eine andere Entfernung zu dem Achsbolzen 10 einnimmt, um damit ergänzend eine besondere Einstellung des angebrachten Prothesenfußes zu ermöglichen.

Wie ersichtlich ergibt sich mit der stufenlos einstellbaren Lage des Exzenters 13 jeweils eine besondere Einstellung des Achsbolzens 9 und damit insgesamt des Knieprothesengelenks 1, womit dieses an alle möglichen notwendigen Einstellungen hinsichtlich Stand und Bewegung sowie Anpassung an irgendwelche Kleidungsstücke anpassbar ist.

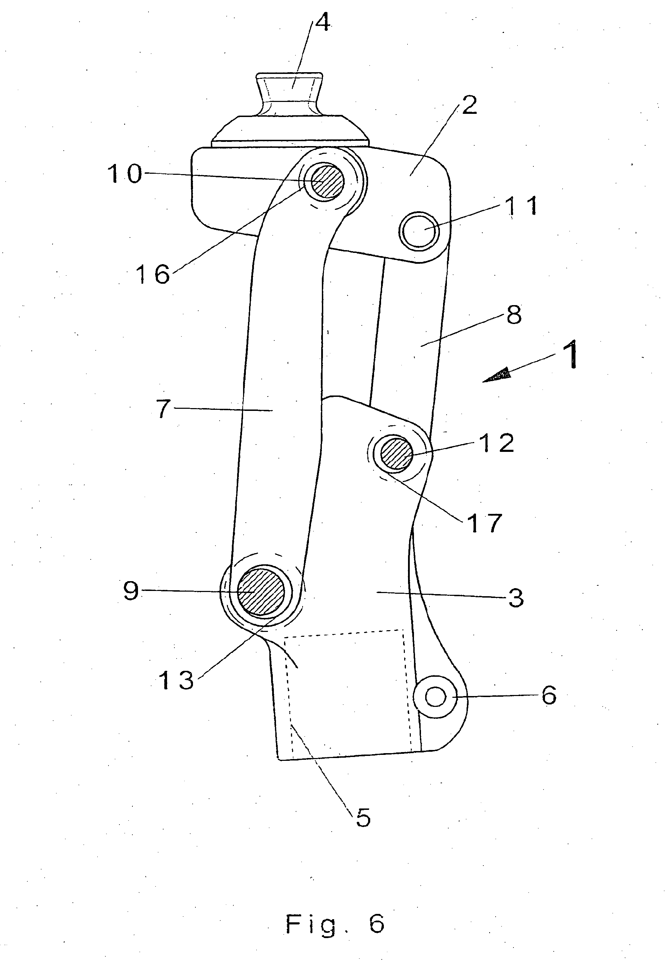

In der Figur 6 ist eine Anordnung des Knieprothesengelenks gemäß Figur 2 in einer Abwandlung dargestellt, bei der außer einem Exzenter in dem Achsbolzen 9 auch die Achsbolzen 10 und 12 mit einem Exzenter 16 und 17 versehen sind. Damit wird dem Knieprothesengelenk eine weitergehende Anpassungsmöglichkeit an verschiedene notwendige Stellungen gegeben.

## Patentansprüche

1. Knieprothesengelenk (1) mit vier Achsbolzen zur Verbindung von vier Gelenkgliedern (2, 3, 7, 8), die jeweils an ihren Enden in je einem Achsbolzen (10, 11, 12, 9) gelagert sind, wobei zwei gegenüberliegende, quer verlaufende Gelenkglieder (2, 3) einerseits mit einem Prothesenschaft und andererseits mit einem Prothesenfuß verbindbar sind und die beiden anderen Gelenkglieder als Längsgelenkglieder (7, 8) jeweils in der Standlage und der Beugelage im Wesentlichen aus einer der Parallellage angenäherten Winkellage in eine demgegenüber stärkere Schräglage zueinander verschwenkbar sind, **dadurch gekennzeichnet, dass** mindestens einer der Achsbolzen (9) des mit dem Prothesenfuß verbundenen Gelenkgliedes (3) als verdrehbarer, feststellbarer Exzenter (13) ausgebildet ist, bei dessen Verdrehung sich das betreffende Längsgelenkglied (7) zu dem Gelenkglied (3) verschiebt und sich der Abstand zwischen den Achsbolzen (9, 12) ändert.

2. Knieprothesengelenk nach Anspruch 1, **dadurch gekennzeichnet, dass** mehrere Achsbolzen als Exzenter (9, 16, 17) ausgebildet sind.

## Claims

1. A prosthetic knee joint (1) comprising four axle pins for connecting four joint members (2, 3, 7, 8) which have each of their ends mounted on a respective axle pin (10, 11, 12, 9) each, of which two horizontal joint members (2, 3) are located opposite each other and can be connected to a prosthetic stem and to a prosthetic foot, respectively, and the other two joint members are longitudinal joint members (7, 8) and can be pivoted relative to each other, both in the upright position and in the bent position, from an angular position, in which they are almost in parallel, to a relatively more inclined position **characterized in that** at least one of said axle pins (9) of the joint member (3) connected to the prosthetic foot is formed as a twistable and lockable eccentric (13) which - when twisted - will cause the respective longitudinal joint member (7) to be displaced toward said joint member (3), resulting in a change of the distance between the axle pins (9,12).

2. The prosthetic knee joint of claim 1 **characterized in that** several axle pins are provided in the form of eccentrics (9, 16, 17).

## Revendications

1. Articulation de genou prothétique (1) avec quatre axes de pivotement de quatre éléments d'articulation (2, 3 7, 8) dont les extrémités sont chacune logées dans un axe de pivotement (10, 11, 12, 9), deux éléments d'articulation (2, 3) situés un à l'opposés de l'autre et s'étendant transversalement pouvant être reliés d'un côté à une tige de prothèse, et de l'autre à un pied de prothèse; les deux autres éléments d'articulation peuvent, en tant qu'éléments d'articulation longitudinaux (7, 8), autant dans la position dépliée que pliée et essentiellement dans une position angulaire se rapprochant de la position parallèle, pivoter l'un par rapport dans une position inclinée plus forte que cette dernière, **caractérisé en ce qu'**au moins l'un des axes de pivotement (9) de l'élément d'articulation (3) relié au pied de prothèse est formé comme un excentrique (13) pivotant et blocable, dont la rotation fait en sorte de déplacer l'élément d'articulation longitudinal (7) en direction de l'élément d'articulation (3) et de modifier l'écart entre les axes de pivotement (9, 12).

2. Articulation de genou prothétique selon la revendication 1, **caractérisé en ce que** plusieurs axes de pivotement sont formés comme excentriques (9, 16, 17).
